# EUROPEAN PATENT APPLICATION

(11) **EP 2 428 201 A1**
(43) Date of publication of application: **14.03.2012**
(21) Application number: 10175833.2
(22) Date of filing: 08.09.2010
(51) Int. Cl.: A61K 9/00, A61K 31/7052, A61K 31/7068, A61K 31/7072, A61K 31/7076

(54) **Oral administration of nucleoside monophosphates**

(71) Applicant: Merck Serono S.A., 1267 Coinsins (CH)
(72) Inventor: The designation of the inventor has not yet been filed
(74) Representative: Merck Serono SA - Geneva Intellectual Property

(57) **Abstract**

The invention relates to the oral pharmaceutical compositions comprising the nucleosides in form of their monophosphates.

## Description

### Field of the Invention

This invention relates to oral compositions comprising nucleosides in form of their monophosphates.

### Description of Related Art

Certain nucleoside drugs or nucleoside analogues, in the following "nucleosides" have been found to have very useful clinical pharmacological properties. These include, but are not limited to, 2'deoxycoformycin (also referred to as dCF, pentostatin), an inhibitor of adenosine deaminase; 2-chloro-2'-deoxyadenosine (also known as cladribine or 2CDA) a drug also resistant to adenosine deaminase through introduction of a chlorine at the 2 carbon. Other nucleosides that have useful pharmacological activity include deoxyadenosines generally, including 2'-deoxyadenosine, 3'-deoxyadenosine, dideoxyadenosine and also didanosine, vidarabine, cytarabine, zalcitabine, stavudine, telbivudine, zidovudine (azidothymidine, or azt), idoxuridine, trifluridine, gemcitabine, nelarabine and clofarabine.

A problem with administering many of these nucleosides relates to the way of administration. Currently, these drugs are mostly used as injectables. As one of the exceptions, cladribine is also available in an oral dosage form in the treatment of multiple sclerosis (N. Engl. J. Med. 2010; 362:416-26). Also clofarabine has been used orally (J. Clin. Oncol. 28:2755-2760).

The art recognizes serious problems with the development of an oral dosage form of nuclosides. Among these is that they have been known for years to be susceptible to acid-catalyzed glycosidic cleavage (e.g. J Pharm Sci. 2000 Apr;89(4):443-56). Therefore, one of skill in the art would expect that an orally administered nucleoside would be cleaved in the stomach, and rendered inactive. It is also known that many nucleosides are subject to glycosidic cleavage by enzymes, such as nuceoside phosphorylases, specifically purine nucleoside phosphorylase (PNP) and pyrimidine nucleoside phosphorylases (PYNP). In particular, it is known that many nucleosides may be cleaved by bacterial phosphorylase, in particular PNP and PYNP, such as that of E. Coli (Eur. J. Biochemistry 233, 886-890, 1995). Indeed, it has been found e.g. that cladribine when administered orally, has a reduced bioavailability as compared to the drug given by injection. For some nucleosides, also the intra-and inter-individual variability of the bioavailability may be affected by oral administration (BMC Pharmacol. 2005; 5: 4).

Various solutions to the problem of reduced bioavailability and/or its variability have been proposed, which suggest protective measures against stomach acid decomposition of the drugs, such as enteric coating of dosage forms or addition of buffering agents or drugs that inhibit release or production of stomach acid (e.g. EP1126828, EP524579).

Although these measures may be effective to a certain degree, there is still a need to further improve the oral dosage forms containing nucleosides in order to achieve an even better bioavailability and/or lower intra- and inter-individual variability thereof.

### Summary of the invention

The disadvantages of the prior art can be addressed by using oral formulations that contain the nucleosides in form of their respective monophosphates, i.e. in form of the respective nucleotides or orthophosphoric acid esters. According to the present invention, these monophosphates are formulated preferably with one or more components which inhibit or reduce the extent of the cleavage of the glycosidic bond and/or phosphoric ester bond of the monophosphates in the acidic environment of the stomach.

### Detailed description of the invention

The present invention relates to oral pharmaceutical formulations that contain the nucleosides in form of their phosphates. According to the invention, the nucleoside preferably bears only a singe phosphate group. Therefore monophosphates of nucleosides are preferred. The preferred monophosphates also only bear a single phosphoric acid ester bond, i.e. that to the nucleoside moiety. Preferably, the phosphate group bears no additional ester bond, i.e. is a monoester, with two acidic OH groups, which may also form a salt with a base, such as alkalihydroxide, to result in the respective mono- or di-alkali phosphates. Preferably, the mono- or di-alkali phosphates are obtained as well known in the art e.g. by use of an appropriate ion exchange resin. The nucleoside monophosphates act as prodrugs that are transformed into the respective nucleoside after oral administration. Bioavailability comparisons are therefore preferably based on nucleosides administered as such and nucleosides that are released from the respective nucleoside monophosphates according to this invention.

Preferred nucleosides employed in the invention are non-naturally occurring, i.e. chemically modified, pyrimindine and purine nuclosides with therapeutic effect and in particular those that have a solubility in water preferably about 1 mg/ml or higher, about 1.5 mg/ml or higher, about 2 mg/ml or higher, about 2.5 mg/ml or about 3 mg/ml or higher (at room temperature, preferably around 20°C, at from about pH 6 to pH 8). The preferred nucleosides contain a non-fluorinated nucleobase, i.e. the nucleobase of the nucleoside, which is linked via a glycosidic bond to the sugar-moiety, does not contain a fluorine atom. More preferably, the nucleosides are purine nucleosides, and in particular those that are susceptible to cleavage of its glycosidic bond by PNP, more specifically bacterial PNP.

Most preferably, the following nucleosides are used according to the invention in form of their monophosphates:
2'deoxycoformycin, 2-chloro-2'-deoxyadenosine (also known as cladribine or 2CDA, 2'-deoxyadenosine, 3'-deoxyadenosine, dideoxyadenosine, didanosine, vidarabine, cytarabine, zalcitabine, stavudine, telbivudine, zidovudine (azidothymidine, or azt), idoxuridine, gemcitabine, nelarabine and clofarabine.

The most preferred nucleoside used according to the invention is cladribine.

Although it is possible that the phosphate group is linked to any of the hydrogen bearing oxygen atoms in the sugar moiety of the nucleoside, the monophosphate group is preferably linked via the 5'-oxygen atom. Thus, nucleoside 5'-monophosphates are particularly preferred.

In one embodiment of the invention, the nucleosides are used preferably in form of the monophosphates of formula I: 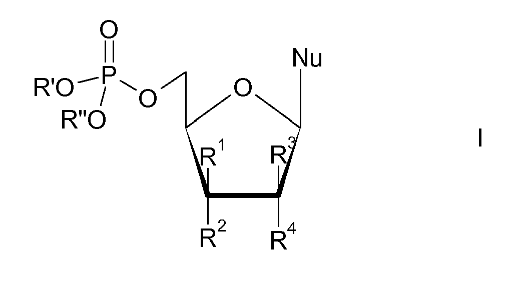 wherein
Nu is a natural nucleobase group or a nucleobase derivative, which is linked via one of its N atoms to the rest of formula I, thereby replacing an H atom at the respective N atom. Preferably, this nucleobase or nucleobase derivative is non-fluorinated. More preferably, it is adenosine, cytosine, thymine, guanine, uracil, 2-chloro-adenosine, 5-iodo-1,2,3,4-tetrahydropyrimidine-2,4-dione (5-iodouracil) or 3,4,7,8-tetrahydroimidazo[4,5-d][1,3]diazepin-8-ol.
R' and R" are, independently from one another, each a counter-ion, preferably H, Na, K, Mg, Ca or NH₄.
R¹, R², R³, R⁴, are independently from one another, H, OH, F or N₃ or one of R¹ and R² form a bond with one of R³ and R⁴.

The preferred nucleoside monophosphates used according to the invention are:
2'deoxycoformycin 5'-monophosphate, 2-chloro-2'-deoxyadenosine 5'-monophosphate (cladribine 5'-monophosphate, 2CDA 5'-monophosphate or CDAMP), 2'-deoxyadenosine 5'-monophosphate, 3'-deoxyadenosine 5'-monophosphate, dideoxyadenosine 5'-monophosphate, didanosine 5'-monophosphate, vidarabine 5'-monophosphate, cytarabine 5'-monophosphate, zalcitabine 5'-monophosphate, stavudine 5'-monophosphate, telbivudine 5'-monophosphate, zidovudine 5'-monophosphate, idoxuridine 5'-monophosphate, gemcitabine 5'-monophosphate, nelarabine 5'-monophosphate and clofarabine 5'-monophosphate, as well as their respective salts, such as the mono- and di-alkali salts, preferably mono- and di-sodium salts.

The most preferred nucleoside monophosphate used according to the invention is 2-chloro-2'-deoxyadenosine 5'-monophosphate (also known as cladribine 5'-monophosphate, 2CDA 5'-monophosphate or CDAMP.

Depending on the pH conditions, the phosphate group may be fully or partially protonated and/or carry a different counter-ion. Preferred counter-ions are sodium, potassium, calcium, magnesium, aluminium, ammonium or mono-, di-, tri- or tetraalkylammonium ions, wherein alkyl is preferably methyl or ethyl. In a preferred embodiment, the nucleoside monophosphates are used in form of their alkali salts, particularly their sodium salts. Thus, the nucleoside monophosphates are preferable mono- or di-alkali salts, such as mono- or di-sodium salts. A very preferred nucleoside monophosphate is 2-chloro-2'-deoxyadenosine 5'-mono-phosphoric acid, mono-sodium 2-chloro-2'-deoxyadenosine 5'-monophosphate, di-sodium 2-chloro-2'-deoxyadenosine 5'-monophosphate.

The nucleoside monophosphates used in the present invention are well known intermediates in the transformation of the respective nucleosides in the human body (Clin. Cancer Res. 1998 Mar;4(3):653-8).

Also, an example of a nucleoside monophosphate for use as a drug has been described in the prior art: Fludarabine is a nucleoside drug containing a fluorinated nucleobase (Fludara®). Fludarabine is only used in form of its monophosphate for injection and oral administration. This drug has also been proposed to be used orally as its monophosphate in a formulation containing an enteric coating (EP1126828).

Fludarabine monophosphate has been originally developed as an injectable drug. This requires a high solubility of the active ingredient in order to be dissolved in a small volume of the liquid for injection. As the free nucleoside fludarabine has a limited solubility under physiological conditions, a change to a more soluble form was required. Phosphorylation is known to result in prodrugs with improved solubility (Advanced Drug Delivery Reviews Volume 19, Issue 2, 22 May 1996, Pages 115-130). Thus, fludarabine is used in form of its monophosphate for administration by injection.

Nevertheless, it is recognized that phosphates of nucleosides are largely not suitable to cross cell membranes, due to the charge they carry under physiological conditions. The nucleoside monophosphate usually needs to be de-phosphorylated before an efficient transfer through a cell membrane can occur. In the case of fludarabine monophosphate, the fact that drug substance needs to be cleaved during the metabolic process, and also requires a more complex synthesis, is balanced by the advantage of having only a small volume to inject.

A subsequently developed oral dosage form also contains the well-known and readily available fludarabine monophosphate (Drug Design, Development and Therapy 2009:3 241-252). There is however no information in the prior art that the use of nucleoside monophosphates in oral formulations would lead to any advantage as compared to the oral use of the respective free nucleosides. This is especially true, since the nucleoside drugs are usually soluble enough to be dissolved in therapeutic amounts in the comparatively large volume of the stomach. For this reason and because the synthesis of the phosphates is requiring additional steps for linking the phosphate to the nucleoside and the nucleoside phosphate also has to be cleaved before uptake by the digestive system, neither the use of nucleoside phosphates nor in particular the use of nucleoside monophosphates for oral administration is encouraged or thought in the prior art to solve the problem of reduced bioavailability and/or variability thereof.

Therefore, there is no information in the prior art that would provide a teaching to improve of the oral dosage forms for delivery of nucleosides, as suggested in the present invention, in order to achieve a better bioavailability and/or lower intra- and inter-individual variability of the bioavailability.

Without wishing to be bound by theory, it may be assumed that the advantageous effects resulting from practicing the present invention may involve the limitation of the extent of cleavage of the glycosidic bond of the nucleosides by e.g. PNP and/or PYNP of enteric bacteria before absorption. Since the nucleosides are provided according to the invention in form of their monophosphates, hydrolysis of the phosphoric ester bond of the nucleoside monophosphates has to occur first in order to release the free nucleosides that are a substrate of PNP and/or PYNP. Such hydrolysis may preferably occur by membrane bound phosphatases, such as alkaline phosphatase, in the brush border of the intestine. Due to preferred absorption of the nucleosides at the enteric membrane directly after hydrolysis, it is assumed that no significant concentration of free nucleosides occurs in the lumen of the intestine, i.e. other than at the membrane layer. In contrast, enteric bacteria are believed to be relatively homogeneously distributed in the lumen of the intestine and not concentrated at the intestinal membrane. This results in a limitation of metabolism of the nucleoside by bacteria and thereby to a limitation of the cleavage of the glycosidic bond. A further improvement can be achieved according to the invention, if the nucleoside monophosphates are formulated with components, which inhibit or reduce the extent of cleavage of the glycosidic bond and/or phosphoric ester bond of the monophosphates in the acidic environment of the stomach. It is assumed that this additional improvement of nucleoside delivery not only results from the preservation of the glycosidic bond from acid cleavage in the stomach, but also from preservation of the phosphoric ester bond until its cleavage occurs by membrane bound phosphatases, such as alkaline phosphatase. As described above, the intact phosphoric ester bond is assumed to be at least partly responsible for a limitation of the cleavage of the glycosidic bond of the respective nucleosides. Thus, by protecting the phosphoric ester bond during stomach transit, also the glycosidic bond may be less likely to be cleaved.

The nucleoside monophosphates used in to the present invention can be manufactured according to or in analogy to known procedures (US 5,602,246, US 4,357,324 , Nucleic Acid Symposium Series No. 52, 583-584, 2008 and J. Med. Chem., 2009, 52 (23), pp 7669-7677). In brief, the nucleoside is preferably phosphorylated as follows: The nucleoside is mixed with phosphorous oxychloride in an alkyl phosphate such as triethylphosphate, or trimethylphosphate, followed by hydrolysis to yield the respective nucleoside 5'-monophosphate after purification. Novel isolated nucleoside 5'-monophosphates are also object of the present invention.

In some cases, it may be appropriate to administer the nucleoside monophosphates in the form of their therapeutically acceptable salts. These salts may be prepared in the conventional manner. Preferably, the salt forms, such as the mono- or di-sodium salts of the nucleoside 5'-monophosphates are obtained by ion exchange using an appropriate resin. To change the counterion e.g. to Na+, the nucleoside 5'-monophosphates are passed over a cation exchange column, such as Bio-Rad AG-50, that had been charged with Na+.

Should the nucleoside monophosphates be used in the form of their salts, the salt former, such as alkali hydroxide, and preferably sodium hydroxide, may also be used in excess, i. e. in an amount greater than equimolar.

Various dosage forms of nucleoside monophosphates may be used in the practice of the invention. For example, the nucleoside monophosphates may be mixed with a pharmacologically acceptable liquid and swallowed. The nucleoside monophosphates also may be compounded into tablets, capsules, pills, lozenges, etc. using conventional compounding techniques.

In an aspect, the invention relates to compositions comprising nucleoside monophosphates, wherein the composition is in a solid dosage form comprising a pill, capsule, or tablet.

Furthermore, the nucleoside monophosphates may be administered or coadministered with conventional pharmaceutical excipients and additives. Preferred formulations and excipents and methods for the preparation of the formulations are disclosed in WO 03053418.

Administration within the context of this invention may be taken to mean administration, coadministration, or both. Coadministration in the context of this invention may be defined to mean the administration of more than one therapeutic in the course of a coordinated treatment to achieve an improved clinical outcome. Such coadministration may also occur during overlapping periods of time.

According to the present invention, the nucleoside monophosphates can be formulated with conventional ingredients used to obtain oral formulations, particularly solid oral formulations, such as tablets.

The dosage form used in the invention may additionally comprise one or more pharmaceutically acceptable inert excipients of which one or more are compression excipients, such as diluents, for example microcrystalline cellulose, disintegrants, for example, crospovidone, and lubricants, for example, magnesium stearate. In a specific embodiment, said compression excipients are present in the dosage form of the invention in an amount between 40% and 80% by weight with respect to the total weight of the dosage form.

Further ingredients include, but are not limited to, gelatin, natural sugars such as raw sugar or lactose, lecithin, pectin, starches (for example corn starch or amylose), dextran, cyclodextrin, preferably beta hydroxypropyl cyclodextrin, polyvinyl pyrrolidone, polyvinyl acetate, gum arabic, alginic acid, xylose, talcum, lycopodium, silica gel (for example colloidal), cellulose, cellulose derivatives (for example cellulose ethers in which the cellulose hydroxy groups are partially etherified with lower saturated aliphatic alcohols and/or lower saturated, aliphatic oxyalcohols, for example methyl oxypropyl cellulose, methyl cellulose, hydroxypropyl methyl cellulose, hydroxypropyl methyl cellulose phthalate), fatty acids as well as magnesium, calcium or aluminum salts of fatty acids, in particular saturated (for example stearates), emulsifiers, oils and fats, in particular vegetable (for example, peanut oil, castor oil, olive oil, sesame oil, cottonseed oil, corn oil, wheat germ oil, sunflower seed oil, cod liver oil, in each case also optionally hydrated); glycerol esters and polyglycerol esters of saturated fatty acids
and their mixtures, it being possible for the glycerol hydroxy groups to be totally or also only partly esterified (for example mono-, di-and triglycerides); pharmaceutically acceptable mono-or multivalent alcohols and polyglycols such as polyethylene glycol and derivatives thereof, esters of aliphatic saturated or unsaturated fatty acids, monovalent aliphatic alcohols, such as ethanol or multivalent alcohols such as glycols, glycerol, diethylene glycol, pentacrythritol, sorbitol, mannitol and the like, which may optionally also be etherified, esters of citric acid with primary alcohols, acetic acid, urea, benzyl benzoate, dioxolanes, glyceroformals, tetrahydrofurfuryl alcohol, polyglycol ethers, dimethylacetamide, lactamides, lactates, ethylcarbonates, silicones (in particular medium-viscous polydimethyl siloxanes), calcium carbonate, sodium carbonate, calcium phosphate, sodium phosphate, magnesium carbonate and the like.

Other auxiliary substances that may be used are those which cause disintegration (so-called disintegrants), such as: cross-linked polyvinyl pyrrolidone, sodium carboxymethyl starch, sodium carboxymethyl cellulose or microcrystalline cellulose. Conventional coating substances may also be used to produce the oral dosage form. Those that may for example be considered are: polymerizates as well as copolymerizates of acrylic acid and/or methacrylic acid and/or their esters; copolymerizates of acrylic and methacrylic acid esters with a lower ammonium group content (for example EudragitR RS), copolymerizates of acrylic and methacrylic acid esters and trimethyl ammonium methacrylate (for example EudragitR RL); polyvinyl acetate; fats, oils, waxes, fatty alcohols; hydroxypropyl methyl cellulose phthalate or acetate succinate; cellulose acetate phthalate, starch acetate phthalate as well as polyvinyl acetate phthalate, carboxy methyl cellulose; methyl cellulose phthalate, methyl cellulose succinate,-phthalate succinate as well as methyl cellulose phthalic acid half ester; ethyl cellulose as well as ethyl cellulose succinate; shellac, gluten; ethylcarboxyethyl cellulose; ethacrylate-maleic acid anhydride copolymer; maleic acid anhydride-vinyl methyl ether copolymer; styrol-maleic acid copolymerizate; 2-ethyl-hexyl-acrylate maleic acid anhydride; crotonic acidvinyl acetate copolymer; glutaminic acid/glutamic acid ester copolymer; carboxymethylethylcellulose glycerol monooctanoate; cellulose acetate succinate; polyarginine.

Plasticizing agents that may be considered as coating substances are:
Citric and tartaric acid esters (acetyl-triethyl citrate, acetyl tributyl-, tributyl-, triethyl-citrate); glycerol and glycerol esters (glycerol diacetate,-triacetate, acetylated monoglycerides, castor oil); phthalic acid esters (dibutyl-, diamyl-, diethyl-, dimethyl-, dipropyl-phthalate), di- (2-methoxy- or 2-ethoxyethyl)phthalate, ethylphthalyl glycolate, butylphthalylethyl glycolate and butylglycolate; alcohols (propylene glycol, polyethylene glycol of various chain lengths), adipates (diethyladipate, di- (2-methoxy- or 2-ethoxyethyl)-adipate; benzophenone; diethyl-and diburylsebacate, dibutylsuccinate, dibutyltartrate; diethylene glycol dipropionate; ethyleneglycol diacetate, -dibutyrate,-dipropionate; tributyl phosphate, tributyrin; polyethylene glycol sorbitan monooleate (polysorbates such as Polysorbar 50); sorbitan monooleate.

It is also possible to add preservatives, stabilizers, buffer substances, flavor correcting agents, sweeteners, colorants, antioxidants and complex formers and the like. Complex formers which may be for example be considered are: chelate formers such as ethylene diamine tetraacetic acid, nitrilotriacetic acid, diethylene triamine pentacetic acid and their salts.

It may optionally be necessary to stabilize the nucleoside monophosphate with physiologically acceptable bases or buffers to a pH range of approximately 6 to 9. Phosphate buffers are preferred.

In some dosage forms, it may be useful to include antioxidants or preservatives. Antioxidants that may for example be used are sodium sulphite, sodium hydrogen sulphite, sodium metabisulphite, ascorbic acid, ascorbylpalmitate,-myristate,-stearate, gallic acid, gallic acid alkyl ester, butylhydroxyanisol, tocopherols as well as synergists (substances which bind heavy metals through complex formation, for example lecithin, ascorbic acid, phosphoric acid ethylene diamine tetracetic acid, citrates, tartrates). Addition of synergists substantially increases the antioxygenic effect of the antioxidants.

Preservatives that may for example be considered are phydroxybenzoic acid esters (for example lower alkyl esters), sodium benzoate, trichloroisobutyl alcohol, phenol, cresol, benzethonium chloride, chlorhexidine and formalin derivatives.

The various oral dosage forms can be prepared according to conventional procedures. For example, tablets can be prepared according to common tableting procedures. Capsules can be prepared according to conventional encapsulating procedures. Liquid dosage forms may be supplied as a made up vial, or may be supplied in a lyophilized state for dilution just prior to administration. Controlled release dosage forms may be prepared according to the particular dosage form being used, as is discussed in brief above.

The preferred solid oral dosage forms used in the present invention can be obtained by conventional techniques by a process that involves mixing a plurality of units containing a nucleoside monophosphate with one or more compression excipients, and compressing the resulting mixture. Compression can be effected by dry or wet methods.

In another aspect, the invention relates to the composition wherein the nucleoside monophosphate is present in an amount effective to treat hematological malignancies, solid tumors, ischemia, CD4+ and/or CD8 T cell mediated diseases, autoimmune diseases, multiple sclerosis rheumatoid arthritis, Morbus Crohn, inflammatory diseases, stroke, myocardial infarction, and ventricular arrhythmia. In yet another aspect, the invention relates to the composition wherein the nucleoside monophosphate is present in an amount effective to treat leukemia. In still another aspect, the invention relates to the composition wherein the leukemia comprises hairy cell leukemia, and chronic lymphocytic leukemia, chronic T-cell lymphoma, acute myelogenous lymphoma, hairy cell leukemia, or chronic lymphocytic leukemia.

The invention also relates to an oral dosage form comprising a nucleoside 5'-monophosphate different from fludarabine 5'-monophosphate, such as trifluridine 5'-monophosphate, and/or its salts.

The nucleoside 5'-monophosphate are preferably therapeutically active. More preferably, they have therapeutic activity in one or more of the following conditions or diseases: cancer, autoimmune diseases, inflammatory diseases and/or viral infections. Another embodiment relates to an oral dosage form according to the invention, wherein the nucleoside 5'-monophosphate and/or its salts are therapeutically active.

The following embodiments of the invention are also preferred:
An oral dosage form comprising a nucleoside 5'-monophosphate and/or salts thereof, having a solubility in water at pH 6 to pH 8 of about 1 mg/ml or higher. A pharmaceutical formulation comprising a nucleoside 5'-monophosphate and/or salts thereof, having a solubility in water at pH 6 to pH8 of about 1mg/ml or higher, and one or more excipients.
   The oral dosage form according to the invention, wherein the nucleoside 5'-monophosphate is present in an amount effective to treat cancer, autoimmune diseases, inflammatory diseases and/or viral infections.
   The oral dosage form according to the invention, wherein the nucleoside 5'-monophosphate is present in an amount effective to treat leukemia and/or muliple sclerosis.

Another embodiment relates to a method of orally administering a dosage form according to the invention.

Dosage amounts and frequency will vary according to the particular nucleoside monophosphate, oral dosage form, and individual patient characteristics.

In a preferred embodiment, the oral dosage form, when administered, results in a reduced decomposition of the nucleoside monophosphate. In particular, such oral dosage form, when administered, results in a reduced cleavage of the glycosidic bond between the nucleobase and the sugar moiety of the nucleoside monophosphate and/or a reduced cleavage of the phosphoric ester bond of the nucleoside monophosphate.

Preferably, this can be achieved by preventing contact between the nucleoside monophosphate and gastric juice after oral administration.

To prevent contact between the nucleoside monophosphate and gastric juice after oral administration, solid oral pharmaceutical forms can be used that comprise a core containing the the nucleoside monophosphate and an outer layer that provides a gastro-resistant coating, which can be separated by one or more intermediate layers. In a preferred embodiment, these solid oral forms are coated with an enteric coat that prevents or reduces release of the nucleoside monophosphate into the stomach. In a particularly preferred oral dosage form enteric coat predominantly releases the nucleoside 5'-monophosphates only in the small intestine of the gastrointestinal tract (gut) following the stomach passage.

In a further aspect, the invention relates to such compositions and/or oral dosage forms wherein the enteric coating comprises hydroxypropylmethylcellulose phthalate, methacrylic acid-methacrylic acid ester copolymer, such as methyl acrylate-methacrylic acid copolymers , polyvinyl acetate-phthalate (PVAP) and cellulose acetate phthalate (CAP), cellulose acetate succinate, hydroxy propyl methyl cellulose phthalate hydroxy propyl methyl cellulose acetate succinate (hypromellose acetate succinate), sodium alginate and stearic acid. Most polymers that are used for this purpose are polyacids that function by virtue of the fact that their solubility in aqueous medium is pH-dependent, and they require conditions with a pH higher then normally encountered in the stomach. The Opadry® coating system of Colorcon, such as Opadry® II, high performance film coating system and polyvinyl acetate-phthalate (PVAP) coatings as well as cellulose acetate phthalate (CAP) coatings, are particularly preferred, especially when prepared in water and applied at a tablet weight gain of about 3 to 30%, more preferably 90 to 20%, most preferably 10 to 17% .

In a preferred embodiment, the outer layer of the dosage form of the invention comprises an enteric coating that comprises a gastro-resistant polymer, such as a methacrylic copolymer, for example a copolymer formed by methacrylic acid and esters of methacrylic acid, a plasticiser such as triethyl acetate or the like, and one or more pharmaceutically acceptable inert excipients, such as talc. In a specific embodiment, said outer enteric coating is present in the dosage form of the invention in an amount between 4% and 30% by weight with respect to the total weight of the dosage form. The coating can be applied to the tablet according to known processes, e.g. Hogan, J. E. (1995). Modified Release Coatings. In: G. Cole (Ed.) Pharmaceutical Coating Technology (p.435). London: Taylor & Francis Ltd. or Lehmann, K. (1997). Polymethacrylate Coating Systems. In: McGinity J (Ed.) Aqueous Polymeric Coatings for Pharmaceutical Dosage Forms (p.136) New York: Marcel Dekker Inc. The outer layer of the dosage form of the invention can contain, in addition, a colored coating comprising a mixture of colorants and opacifiers together with other pharmaceutically acceptable excipients, such as a mixture of polydextrose, HPMC, titanium dioxide, PEG 400 and colorants. The colored coating layer can be mixed with the outer enteric coating layer to define a single outer layer. In a specific embodiment, said colored layer is present in the dosage form of the invention in an amount between 1.5% and 15% by weight with respect to the total weight of the dosage form.

The preferred dosage form of the invention is resistant to solution in an acid medium, stable when passing through the gastric juice and allows liberating the active principle in an alkaline or neutral aqueous medium, the characteristic conditions of the proximal area of the small intestine. Thus, the preferred dosage form promote the nucleoside monophosphates remaining physically incorporated in the dosage form for a specified period when exposed to gastric juice. Yet the enteric coatings are designed to disintegrate in intestinal fluid for ready absorption.

The enteric coat can be applied to the solid oral dosage forms by means generally known in the art. The tablet coating can e.g. be performed in a vented pan equipped with a spray-gun.

In a further embodiment, the invention also relates to a controlled release formulation containing a nucleoside monophosphate.

There are a number of controlled release drug formulations that are developed preferably for oral administration. These include, but are not limited to, osmotic pressure-controlled gastrointestinal delivery systems; hydrodynamic pressure-controlled gastrointestinal delivery systems; membrane permeationcontrolled gastrointestinal delivery systems, which include microporous membrane permeation-controlled gastrointestinal delivery devices; gastric fluidresistant intestine targeted controlled-release gastrointestinal delivery devices; gel diffusion-controlled gastrointestinal delivery systems; and ion-exchangecontrolled gastrointestinal delivery systems, which include cationic and anionic drugs. Additional information regarding controlled release drug delivery systems may be found in Yie W. Chien, Novel Drug Delivery Systems, 1992 (Marcel Dekker, Inc.). some of these formulations will now be discussed in more detail.

Another type of useful oral controlled release structure is a solid dispersion. A solid dispersion may be defined as a dispersion of one or more active ingredients in an inert carrier or matrix in the solid state prepared by the melting (fusion), solvent, or melting-solvent method. Akihiko Hasegawa, Super Saturation Mechanism of Drugs from Solid Dispersions with Enteric Coating Agents, Chem. Pharm. Bull. 36: 4941-4950 (1998). The solid dispersions may be also called solid-state dispersions.

In still another aspect, the invention relates to the composition wherein the composition comprises a solid dispersion. In yet another aspect, the invention relates to the composition wherein the solid dispersion comprises a water soluble or a water insoluble carrier. In another aspect, the invention relates to the composition wherein the water soluble or water insoluble carrier comprises polyethylene glycol, polyvinylpyrrolidone, hydroxypropylmethylcellulose, phosphatidylcholine, polyoxyethylene hydrogenated castor oil, hydroxypropylmethylcellulose phthalate, carboxymethylethylcellulose, or hydroxypropylmethylcellulose, ethyl cellulose, or stearic acid In still another aspect, the invention relates to the composition wherein the composition is in a dosage form comprising a complex between an ion exchange resin and the nucleoside monophosphate.

Another controlled release dosage form is a complex between an ion exchange resin and a nucleoside monophosphate according to the invention.

In a further embodiment, the nucleoside monophosphates may be administered with various agents to reduce acid concentration in the stomach. This reduces acid lability and allows for enhanced concentrations of nucleoside monophosphate for enhanced gastric and/or intestinal absorption. For example, the nucleoside monophosphate may be coadministered with an H2 inhibitor such as cimetidine, an acid neutralizer such as calcium carbonate or magnesium carbonate, or a proton pump inhibitor. Preferred is furthermore the coadministration with omeprazole. Also, a combination or a coadministration of the agents to reduce acid concentration in the stomach is preferred. In another embodiment, the problem of instability of the nucleoside monophosphates, due to stomach acid is addressed by creating an alkaline environment around the nucleoside monophosphate. This is achieved by using alkaline salts thereof and/or by incorporating an alkaline reacting compound in the gastro-resistant pharmaceutical preparation.

The invention also relates to methods of preparation of the dosage forms comprising the steps of
a) phosphorylating a nucleoside according to the invention
   and
b) formulating the resulting nucleoside 5'-monophosphate into an oral dosage form
   and optionally
c) coating the oral dosage form with an enteric coat.

Dosage amounts and frequency will vary according to the particular nucleoside monophosphate, oral dosage form, and individual patient characteristics.

Generally speaking, determining the dosage amount and frequency for a particular nucleoside monophosphate, oral dosage form, and individual patient characteristic can be accomplished using conventional dosing studies, coupled with appropriate diagnostics. In preferable embodiments, the dosage frequency ranges from daily to monthly doses, more preferably biweekly doses. In preferred embodiments the dosage of the nucleoside monophosphate is administered according to known dosage regimen adapted for the improved bioavailability and pharmacokinetic parameters provided by the present invention. In a preferred embodiment, the oral dosage amount and frequency of cladribine 5'-monophosphate in the treatment of multiple sclerosis according to WO 2006/067141 can be adapted for the improved bioavailability and pharmacokinetic parameters provided by the present invention.

### Examples:

### Example 1

### Preparation of nucleoside 5'-monophosphates

First, a Triethylammonium Hydrogen Carbonate Buffer (TEAB) is prepared in a 1 M solution, by bubbling CO₂ through an aqueous 1 M triethylamine solution at 0-4 °C for several hours, resulting in a pH of approximately 7.4-7.6. The solution may be appropriately diluted for use.

The respective nucleoside (1 mmol) is dissolved in 5 ml of trimethyl phosphate. The stirred mixture is cooled to 4 °C under argon atmosphere and dry 1,8-bis(dimethylamino)naphthaline (0.32 g, 1.5 mmol) is added. After 5 minutes, 0.20 g (1.3 mmol) POCI₃ is added. The mixture is stirred for 5 h at 0-4 °C and subsequently poured into 10 ml of a stirred 0.5 M aqueous TEAB solution described above (0-4 °C and pH 7.5). The solution is stirred for 1 h whilst reaching room temperature and than extracted with tert.-butylmethyl ether to remove the trimethylphosphate. The aqueous phase is lyophilized to yield the respective nucleoside 5'-monophosphate, for further purification. The progress of the reactions can be followed by thin layer chromatography (TLC) with e.g. detection of ultraviolet (UV) absorption, using e.g. aluminum sheets with silica gel 60 F254 (Merck). A mixture of 2-propanol:NH₄OH (25% of ammonia in water):water = 6:3:1 can be used for the TLC.

Ion exchange chromatography can be used to purify the nucleoside 5'-monophosphates (e.g. using an FPLC instrument such as KTA FPLC, Amersham Biosciences, with e.g. an XK 26 mm/20 cm column, Pharmacia, using e.g. Sephadex DEAE A-25 gel hydrogen carbonate-form swelled in a 1 M solution of TEAB at 4 °C). The nucleoside 5'-monophosphate to be purified are dissolved in 2 ml of aqueous triethylammonium hydrogen carbonate buffer and subjected to chromatography on a column equilibrated with deionized water. The product is eluted with water, followed by a solvent gradient of water (TEAB 1 M buffer from 0 to 100%). Approximately 500 ml of solvent is used to elute the nucleoside 5'-monophosphate. The fractions containing the nucleoside 5'-monophosphate are pooled and lyophilized.

Instead or in addition to the ion exchange chromatography, the lyophilized nucleoside 5'-monophosphate can be purified using preparative HPLC wherein the nucleoside 5'-monophosphate is dissolved in 5 ml of deionized water and injected into an RP-HPLC column (e.g. Knauer 20 mm ID, Eurospher-100 C18). A solvent gradient is applied as follows to elute the nucleoside 5'-monophosphate: 0-50% of acetonitrile in 50 mM aqueous NH₄HCO₃ buffer for 40 min at a flow rate of 5 ml/min. UV absorption at 254 nm can be used to detect the product. The fractions containing the nucleoside 5'-monophosphate are pooled and repeatedly lyophilized to remove the NH₄HCO₃ buffer. A salt form, such as the mono- or di-sodium salt of the nucleoside 5'-monophosphate is obtained by ion exchange using an appropriate resin. To change the counter ion e.g. to Na+, the nucleoside 5'-monophosphate dissolved in water is passed over a cation exchange column, such as Bio-Rad AG-50, that had been charged with Na+. Subsequent lyophilization results in the respective salt in solid form.

The following preferred nucleoside 5'-monophosphates, as well as their respective salts, such as the mono- and di-alkali salts, preferably mono- and di-sodium salts, can be prepared from the respective nucleosides according to the above described procedure:
2'deoxycoformycin 5'-monophosphate from 2'deoxycoformycin, 2-chloro-2'-deoxyadenosine 5'-monophosphate (cladribine 5'-monophosphate, 2CDA 5'-monophosphate or CDAMP) from 2-chloro-2'-deoxyadenosine, 2'-deoxyadenosine 5'-monophosphate from 2'-deoxyadenosine, 3'-deoxyadenosine 5'-monophosphate from 3'-deoxyadenosine, dideoxyadenosine 5'-monophosphate from dideoxyadenosine, didanosine 5'-monophosphate from didanosine, vidarabine 5'-monophosphate from vidarabine, cytarabine 5'-monophosphate from cytarabine, zalcitabine 5'-monophosphate from zalcitabine, stavudine 5'-monophosphate from stavudine, telbivudine 5'-monophosphate from telbivudine, zidovudine 5'-monophosphate from zidovudine, idoxuridine 5'-monophosphate from idoxuridine, gemcitabine 5'-monophosphate from gemcitabine, nelarabine 5'-monophosphate from nelarabine and clofarabine 5'-monophosphate from clofarabine.

For further use in oral dosage forms, the specified amounts of nucleoside 5'-monophosphates may be adjusted to about pH 7.4 by sodium hydroxide and/or diluted phosphate buffer and used as such or may subsequently be lyophilized.

### Example 2:

10 mg of cladribine in form of its 5'-monophosphate obtained in example 1, are compounded with 60 mg microcrystalline cellulose (Avicel), 75 mg lactose monohydrate, 0.8 mg colloidal anhydrous silicon dioxide, 3 mg croscarmellose sodium (sodium carboxymethylcellulose) and 1.5 mg magnesium stearate. This mixture is tableted using a conventional tableting press. The resulting tablet is then coated with an aqueous supension of 2.3 mg hydroxypropylmethylcellulose, 0.5 mg talcum and 1.2 mg titanium dioxide.

### Example 3:

10 mg of cladribine in form of its 5'-monophosphate obtained in example 1, are compounded with 60 mg microcrystalline cellulose (Avicel), 75 mg lactose monohydrate, 0.8 mg colloidal anhydrous silicon dioxide, 3 mg croscarmellose sodium (sodium carboxymethylcellulose) and 1.5 mg magnesium stearate. This mixture is tableted using a conventional tableting press. The resulting tablet is then enterically coated with Polyvinyl Acetate Phthalate (Phthalavin® enteric coating polymer, PVAP, Sureteric, Colorcon) dispersed in water (15% w/w). The coat is applied to the tablet to obtain an approximate 10% weight gain. An optional topcoat of Opadry II (Colorcon) dispersed in water (15% w/w) is applied to the tablet to obtain an approximate 2% weight gain.

### Example 4:

10 mg of cladribine in form of its 5'-monophosphate obtained in example 1, are compounded with 60 mg microcrystalline cellulose (Avicel), 75 mg lactose monohydrate, 0.8 mg colloidal anhydrous silicon dioxide, 3 mg croscarmellose sodium (sodium carboxymethylcellulose) and 1.5 mg magnesium stearate. This mixture is tableted using a conventional tableting press. The resulting tablet is then enterically coated with methacrylic acid- methyl methacrylate 1:1 copolymer (Opadry II, Colorcon) dispersed in water (15% w/w). The coat is applied to the tablet to obtain an approximate 15% weight gain.

### Example 5:

5 mg of cladribine in form of its 5'-monophosphate obtained in example 1, are compounded with 60 mg microcrystalline cellulose (Avicel), 75 mg lactose monohydrate, 0.8 mg colloidal anhydrous silicon dioxide, 3 mg croscarmellose sodium (sodium carboxymethylcellulose) and 1.5 mg magnesium stearate. This mixture is tableted using a conventional tableting press. The resulting tablet is then enterically coated with methacrylic acid- methyl methacrylate 1:1 copolymer (Opadry II, Colorcon) dispersed in water (15% w/w). The coat is applied to the tablet to obtain an approximate 15% weight gain.

### Example 6:

The bioavailability of cladribine resulting from a cyclodextrin containing tablet according to Example 3 of WO 2004/087101, Lot FD04, Batch No 126, adjusted to contain 5 mg of cladribine, and an enterically coated tablet containing cladribine 5'-monophosphate according to example 5 above, is evaluated in a beagle dog model. This model is expected to be representative for the human experience. Bioavailability and pharmacokinetic studies are conducted in a beagle dog model as follows:
In a first test period, 5 mg cladribine (0.25 mg/ml in isotonic saline) administered intravenously to male beagle dogs. Serial blood samples are collected over 48 hours. In the second test period, one half of the subjects receives orally a tablet according to example 5, and the other half of the subject receives orally a tablet according WO 2004/087101. Serial blood samples are collected over 48 hours. The third test period repeates the second test period with the exception that the subject previously receiving the tablet according to example 5 are now given the tablet according to WO 2004/087101 and vice versa. Cladribine levels in the blood are measured by HPLC and an LC/MS method. Pharmacokinetic analysis is performed on the basis of individual plasma concentration versus time curves. For calculation of the bioavailability, the oral AUC/dose value is divided by the intravenous AUC/dose value. The individual plasma level-time curves are obtained.
Intravenous values are considered to give 100% bioavailability, and plasma levels for oral forms are compared thereto. Small inter- individual variability can be found after oral administration of the tablet according to example 1.
The use of the tablet according to example 5 translates in a higher bioavailability of cladribine as compared to the tablet according to WO 2004/087101.

### Example 6:

5 mg of pentostatin in form of its 5'-monophosphate obtained in example 1, are mixed with 50 ml of sterile water and 1 mg of sodium saccharide as a sweetener. A phosphoric acid buffer is used to adjust the pH of the solution to about 7.4. The mixture is suitable for oral administration.

### Example 7:

10 mg of pentostatin in form of its 5'-monophosphate obtained in example 1, are compounded with 60 mg microcrystalline cellulose (Avicel), 75 mg lactose monohydrate, 0,8 mg colloidal anhydrous silicon dioxide, 3 mg croscarmellose sodium (sodium carboxymethylcellulose) and 1,5 mg magnesium stearate. This mixture is tableted using a conventional tableting press. The resulting tablet is then coated with an aqueous supension of 2.3 mg hydroxypropylmethylcellulose, 0.5 mg talcum and 1.2 mg titanium dioxide.

### Example 8:

10 mg of pentostatin in form of its 5'-monophosphate obtained in example 1, are compounded with 60 mg microcrystalline cellulose (Avicel), 75 mg lactose monohydrate, 0.8 mg colloidal anhydrous silicon dioxide, 3 mg croscarmellose sodium (sodium carboxymethylcellulose) and 1.5 mg magnesium stearate. This mixture is tableted using a conventional tableting press. The resulting tablet is then enterically coated with methacrylic acid-methyl methacrylate 1:1 copolymer (Opadry II, Colorcon) dispersed in water (15% w/w). The coat is applied to the tablet to obtain an approximate 15% weight gain.

## Claims

1. An oral dosage form comprising a nucleoside 5'-monophosphate of formula I, and/or salts thereof 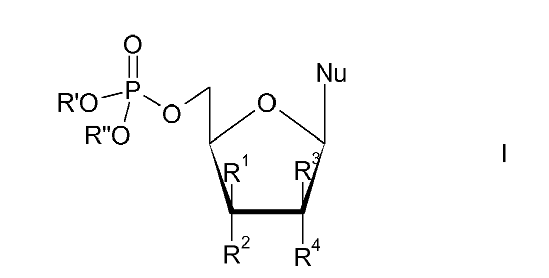 wherein
Nu is a natural nucleobase group or a nucleobase derivative, which is linked via one of its N atoms to the rest of formula I, thereby replacing an H atom at the respective N atom of the nucleobase,
R' and R" are, independently from one another, each a counter-ion,
R¹, R², R³, R⁴, are independently from one another, H, OH, F or N₃ or one of R¹ and R² form a bond with one of R³ and R⁴.

2. An oral dosage form comprising a non-fluorinated nucleoside 5'-monophosphate and/or salts thereof.

3. An oral dosage form comprising one of the following monophosphates:
2'deoxycoformycin 5'-monophosphate, 2-chloro-2'-deoxyadenosine 5'-
monophosphate (cladribine 5'-monophosphate, 2CDA 5'-
monophosphate or CDAMP), 2'-deoxyadenosine 5'-monophosphate,
3'-deoxyadenosine 5'-monophosphate, dideoxyadenosine 5'-
monophosphate, didanosine 5'-monophosphate, vidarabine 5'-
monophosphate, cytarabine 5'-monophosphate, zalcitabine 5'-
monophosphate, stavudine 5'-monophosphate, telbivudine 5'-
monophosphate, zidovudine 5'-monophosphate, idoxuridine 5'-
monophosphate, gemcitabine 5'-monophosphate, nelarabine 5'-
monophosphate and clofarabine 5'-monophosphate, and/or salts
thereof.

4. An oral dosage form according to claims 1 to 3, comprising one or more components to reduce the extent of decomposition of the nucleoside 5'-monophosphate by acid.

5. An oral dosage form according to claim 4, wherein the oral dosage form is solid.

6. An oral dosage form according to claims 4 and 5, wherein the oral dosage form comprises at least one enteric coat.

7. An oral dosage form according to claim 6, wherein the enteric coat comprises one or more of the components selected from the group of hydroxypropylmethylcellulose phthalate, methacrylic acid-methacrylic acid ester copolymer, such as methyl acrylate-methacrylic acid copolymers , polyvinyl acetate-phthalate (PVAP) and cellulose acetate phthalate (CAP), cellulose acetate succinate, hydroxy propyl methyl cellulose phthalate hydroxy propyl methyl cellulose acetate succinate (hypromellose acetate succinate), sodium alginate and stearic acid.

8. Use of an oral dosage form according to the foregoing claims for the preparation of a medicament to be administered orally.

9. Use of an oral dosage form according to the foregoing claims for the preparation of a medicament for the treatment of conditions that are responsive to the nucloside that is contained in the dosage form in form of its 5'- monophosphate.

10. Use of an oral dosage form according to the foregoing claims for the preparation of a medicament for the treatment of cancer, autoimmune diseases, inflammatory diseases and/or viral infections.

11. Use of an oral dosage form according to claim 10 for the preparation of a medicament fort he treatment of leukemia, HIV and/or muliple sclerosis.

12. A pharmaceutical formulation comprising a non-fluorinated nucleoside 5'-monophosphate and/or salts thereof and one or more excipients.

13. A pharmaceutical formulation comprising a nucleoside 5'-phospate of formula I according to claim 1 and one or more excipients.

14. A pharmaceutical formulation comprising 2'deoxycoformycin 5'-monophosphate, 2-chloro-2'-deoxyadenosine 5'-monophosphate (cladribine 5'-monophosphate, 2CDA 5'-monophosphate or CDAMP), 2'-deoxyadenosine 5'-monophosphate, 3'-deoxyadenosine 5'-monophosphate, dideoxyadenosine 5'-monophosphate, didanosine 5'-monophosphate, vidarabine 5'-monophosphate, cytarabine 5'-monophosphate, zalcitabine 5'-monophosphate, stavudine 5'-monophosphate, telbivudine 5'-monophosphate, zidovudine 5'-monophosphate, idoxuridine 5'-monophosphate, gemcitabine 5'-monophosphate, nelarabine 5'-monophosphate and clofarabine 5'-monophosphate, and/or salts thereof and one or more excipients.

15. A method for preparation of an oral dosage form, comprising phosphorylation of a nucleoside to result in the nucleoside 5'-phosphate according to claims 1, 2 or 3, tabletting the nucleoside 5'-phosphate and optionally coating the resulting tablet with an enteric coat.
